# EUROPEAN PATENT APPLICATION

(11) **EP 3 607 953 A1**
(43) Date of publication of application: **12.02.2020**
(21) Application number: 18746066.2
(22) Date of filing: 16.07.2018
(51) Int. Cl.: A61K 31/711, A61K 31/7008, A61K 31/198, A61K 31/737, A61P 19/02, A61P 29/00

(54) **COMPOSITION, COMPRISING DNA FRAGMENT MIXTURE AND MATRIX METALLOPROTEASE PRODUCTION INHIBITOR, FOR PREVENTION AND TREATMENT OF ARTHRITIS**

(30) Priority: 12.06.2018 KR 20180067417
(71) Applicant: BR Pharm Co., Ltd, Wonju-si Gangwon-do 26348 (KR)
(72) Inventor: The designation of the inventor has not yet been filed
(74) Representative: Izquierdo Blanco, Maria Alicia
(86) International application number: PCT/KR2018/008001
(87) International publication number: WO 2019/240323

(57) **Abstract**

The present invention relates to a composition for preventing and treating arthritis, which includes a DNA fragment mixture and a matrix metalloproteinase (MMP) production inhibitor capable of overcoming the problem of MMP production induced when using the DNA fragment mixture in the prevention and treatment of arthritis, thereby being able to inhibit inflammatory pain and to make cartilage regeneration possible. The composition may inhibit matrix metalloproteinase production induced by the DNA fragment mixture, and, thus, may be effectively used without adverse effects for induction of chondrocyte proliferation, repair of damaged chondrocytes, regeneration of cartilage, and relief of arthritis.

## Description

### BACKGROUND

### 1. Technical Field

The present invention relates to a composition for preventing and treating arthritis, which includes a DNA fragment mixture and a matrix metalloproteinase (MMP) production inhibitor capable of overcoming the problem of MMP production induced when using the DNA fragment mixture for the prevention and treatment of arthritis, thereby being able to inhibit inflammatory pain and to make cartilage regeneration possible.

### 2. Description of the Related Art

Arthritis refers to a disease caused by inflammatory changes in the joints due to various factors such as bacteria and trauma. The incidence of arthritis is estimated to be about 30% of the population aged 60 years or over. According to foreign statistics, arthritis is known to be a common disease following heart disease. In addition, according to a recent survey by the Korea Institute for Health and Social Affairs, Korean people aged 50 years or older have been reported to have the highest incidence of arthritis, overtaking diabetes and hypertension.

Therefore, many researchers expect that arthritis will become the most important disease that can interfere with the quality of life of mankind in the 21^{st} century along with cancer or cardiovascular diseases.

Meanwhile, arthritis can be classified into non-inflammatory arthritis and inflammatory arthritis. Non-inflammatory arthritis typically includes osteoarthritis, and inflammatory arthritis typically includes rheumatoid arthritis.

Degenerative arthritis is caused by the following mechanism. When degeneration (such as aging) of chondrocytes in the joint occurs, the synthesis of type II collagen and proteoglycan, which are joint matrices, in the chondrocytes, is inhibited, and at the same time, inflammatory cytokines, such as IL-1β (interlekin-1β) and tumor necrosis factors, are produced. For this reason, the synthesis and activity of matrix metalloproteinase (MMP) that degrades joint matrices are increased in joint cells, thereby destroying joint tissue, causing degenerative arthritis.

Therefore, the purpose of treating arthritis is not only to reduce pain and inflammation in the joint or to inhibit prevent deformation of the joint by inhibiting the activity of MMP, but also to reveal the intracellular action mechanism that causes arthritis more fundamentally and to control the mechanism.

Meanwhile, treatment of degenerative arthritis, which is currently being used clinically, is performed using therapeutic drugs (analgesics, steroidal agents, non-steroidal anti-inflammatory agents, etc.) or cartilage protective agents (hyaluronic acid, glucosamine, chondroitin, etc.), or by surgical treatments (arthroscopic surgery, proximal tibial osteotomy, unicompartmental joint replacement, total knee replacement, etc.).

However, the therapeutic drugs have only the effect of nonspecifically relieving pain or the inflammatory response itself, and the cartilage protective agents only serve to protect the joint by nourishing chondrocytes or alleviating impact on chondrocytes. Moreover, these drugs also show side effects such as weight gain, hypertension induction, peptic ulcer induction, etc.

Referring to Korean Patent Application Publication No. 10-2013-0044195, entitled "Composition for cartilage regeneration including DNA fragment mixture isolated from fish's testis," and Korean Patent Application Publication No. 10-2017-0100236, entitled "Functional health food for prevention or relief of arthritis including DNA fragment mixture," a DNA fragment mixture obtained from fish's testis or the like has effects on chondrocyte proliferation, the repair of damaged chondrocytes, cartilage regeneration, and arthritis relief, and thus is used for the prevention and treatment of arthritis.

### SUMMARY

However, when a DNA fragment mixture is used for the prevention and treatment of arthritis, a problem arises in that it induces the production of matrix metalloproteinase (MMP) that may adversely affect the joint. If this problem is not solved, it may inevitably impair the effect of the DNA fragment mixture on the prevention and treatment of arthritis.

Therefore, it is an object of the present invention to provide a novel composition for preventing and treating arthritis, which overcomes the problem of matrix metalloproteinase (MMP) production induced by a DNA fragment mixture.

To achieve the above object, the present invention provides a composition for preventing and treating arthritis, including a DNA fragment mixture and a matrix metalloproteinase production inhibitor.

In the composition for preventing and treating arthritis according to the present invention, the matrix metalloproteinase production inhibitor is preferably glucosamine or a pharmaceutically acceptable salt thereof.

In the composition for preventing and treating arthritis according to the present invention, the matrix metalloproteinase production inhibitor is preferably N-acetyl-D-glucosamine (NAG) or a pharmaceutically acceptable salt thereof.

In the composition for preventing and treating arthritis according to the present invention, the matrix metalloproteinase production inhibitor is preferably alliin or a pharmaceutically acceptable salt thereof.

In the composition for preventing and treating arthritis according to the present invention, the matrix metalloproteinase production inhibitor is preferably sodium butyrate (SB).

In the composition for preventing and treating arthritis according to the present invention, the matrix metalloproteinase production inhibitor is preferably chondroitin sulfate (CS).

### BRIEF DESCRIPTION OF THE DRAWINGS

The above and other objects, features and advantages of the present invention will be more clearly understood from the following detailed description taken in conjunction with the accompanying drawings, in which:
FIG. 1 is a graph showing the effect of a DNA fragment mixture on the expression of MMP-3 (A) and MMP-13 (B) in SW-1353 cells stimulated with 10 ng/ml of IL-β.
FIG. 2 is a micrograph showing the change in migration of SW-1353 cells by treatment with different concentrations of a DNA fragment mixture. Gap distance: 500 µm.
FIG. 3 depicts a graph and micrograph that show the effect of a DNA fragment on the viability of SW-1353 cells. The value in the graph of FIG. 3A is expressed as mean ± SE (n=3), and the scale bar unit in the image of FIG. 3B is 1000 µm.
FIG. 4 is a graph showing the effect of glucosamine on the expression of MMP-3 and MMP-13 in SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 5 is a graph showing the effect of N-acetyl-D-glucosamine (NAG) on the expression of MMP-3 and MMP-13 in SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 6 is a graph showing the effect of alliin on the expression of MMP-3 and MMP-13 in SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 7 is a graph showing the effect of sodium butyrate (SB) on the expression of MMP-3 and MMP-13 in SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 8 is a graph showing the effect of chondroitin sulfate (CS) on the expression of MMP-3 and MMP-13 in SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 9 depicts images showing a 3D dynamic culture model for SW-1353 cells. A: scheme for 3D dynamic culture of SW-1353 cells; B: top-view image; C: cross-sectional image; scale bar unit in B: 500 µm; and scale bar unit in C: 200 µm.
FIG. 10 depicts images showing the morphology of SW-1353 cells in the 3D dynamic culture model. Scale bar unit: 500 µm.
FIG. 11 depicts images showing the morphology of SW-1353 cells on different gel-cell matrices during 3D culture. Scale bar unit: 200 µm; green (calcein AM): live; red (EthD-1): dead; and blue (Hoechst): nuclei.
FIG. 12 is a graph showing the effect of a DNA fragment mixture on the expression of MMP-3 (A) and MMP-13 (B) in SW-1353 cells, stimulated with IL-β, in 3D 10% serum-containing and serum-free states.
FIG. 13 is a graph showing the effect of a DNA fragment mixture on the expression of MMP-3 (A) and MMP-13 (B) in SW-1353 cells, stimulated with IL-β, in a serum-free state.
FIG. 14 is a graph showing the effect of glucosamine on the expression of MMP-3 and MMP-13 in 3D SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 15 is a graph showing the effect of N-acetyl-D-glucosamine (NAG) on the expression of MMP-3 and MMP-13 in 3D SW-1353 stimulated with 10 ng/ml of IL-β. The concentration of the sample is as follows: 0 µg/ml in A and B; 10 µgml in C and D; 100 µgml in E and F; 1000 µg/ml in G and H.
FIG. 16 is a graph showing the effect of alliin on the expression of MMP-3 and MMP-13 in 3D SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 17 is a graph showing the effect of sodium butyrate (SB) on the expression of MMP-3 and MMP-13 in 3D SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.
FIG. 18 is a graph showing the effect of chondroitin sulfate(CS) on the expression of MMP-3 and MMP-13 in 3D SW-1353 stimulated with 10 ng/ml of IL-β. The concentration of the sample is as follows: 0 µg/ml in A and B; 10 µg/ml in C and D; 100 µg/ml in E and F; 1000 µg/ml in G and H.

### DETAILED DESCRIPTION

The present invention will be described in detail below.

While the present inventors have conducted experiments to reveal the intracellular action mechanism of a DNA fragment as an arthritis therapeutic agent through 2D and 3D *in vitro* human chondrocyte models DNA, the present inventors have found the problem of production of matrix metalloproteinase (MMP), particularly matrix metalloproteinase-13 (MMP-13), induced by the DNA fragment mixture. This finding led to the present invention.

Therefore, the present invention provides a composition for preventing and treating arthritis, including a DNA fragment mixture and a matrix metalloproteinase production inhibitor.

Furthermore, according to the present invention, a 3D dynamic culture model that cultures cells three-dimensionally is used to maximize the prediction of safety and efficiency of the composition for preventing or treating arthritis, making it possible to confirm human body responses in a more accurate manner than a 2D culture method.

A mixture of DNA fragments, also called polydeoxyribonucleotides (PDRN), according to the present invention, is a mixture of low-molecular-weight fragments isolated from the sperm or testis of fish, particularly trout, salmon or the like. These fragments are the essential components of cells, and a mixture thereof is used in various applications, including pharmaceutical drugs intended to treat and alleviate wound sites by injecting them into the wound sites or to treat or relieve arthritis by the cartilage regeneration effect thereof, functional health foods, cosmetic products intended to reduce wrinkles by cell activation, food additives, biochemical experimental materials, and the like.

The DNA fragment mixture according to the present invention is used for the prevention and treatment of arthritis, and the basic uses thereof are already known. The basic effects and mechanisms of the DNA fragment mixture are also applied to the present invention, and for example, reference may be made to: Korean Patent Application Publication No. 10-2013-0044195, entitled "Composition for cartilage regeneration including DNA fragment mixture isolated from fish's testis"; Korean Patent Application Publication No. 10-2017-0100236, entitled "Functional health food for prevention or relief of arthritis including DNA fragment mixture"; PCT International Publication No. WO 2010/049898 A1 "INJECTABLE POLYDEOXYRIBONUCLEOTIDE COMPOSITION FOR THE TREATMENT OF OSTEOARTICULAR DISEASES; published papers "Polydeoxyribonucleotide (PDRN) promotes human osteoblast proliferation: A new proposal for bone tissue repair (Life Sciences 73 (2003) 1973-1983) and "Protective effects of polydeoxyribonucleotides on cartilage degradation in experimental cultures (Cell Biochem Funct 2013; 31: 214-227), and the like.

The molecular weight, viscosity, and the like of the DNA fragment mixture that is used in the present invention are not particularly limited, and a method for preparing the same is also not particularly limited. One example of the preparation method includes: crushing the testis of *Oncorhynchus mykiss* (trout farmed in the sea; referred to as sea trout) with purified water and sodium chloride by means of a crusher; adding distilled water to the crushed testis, and adding sodium dodecyl sulfate (hereinafter referred to as (SDS) thereto, followed by keeping at 100 to 105°C for 4 to 5 hours in order to reduce the molecular weight of the testis; cooling the heated testis to room temperature, followed by centrifugation at 15,000 rpm, and removing testis debris; and filtering the residue, and adding the filtrate slowly to ethanol, thereby obtaining a white DNA fragment mixture. Before use, the white DNA fragment mixture may be dewatered with ethanol or anhydrous ethanol, filtered, and dried, so that the purity thereof can be increased.

The matrix metalloproteinase production inhibitor is not particularly limited and may be any widely known in the art to which the present invention pertains. Examples thereof include glucosamine or a pharmaceutically acceptable salt thereof, N-acetyl-D-glucosamine (NAG), alliin, sodium butyrate (SB), and chondroitin sulfate (CS).

Hereinafter, the present invention will be described in detail through experiments on the DNA fragment mixture, and glucosamine, N-acetyl-D-glucosamine (NAG), alliin, sodium butyrate (SB) and chondroitin sulfate (CS), which are matrix metalloproteinase production inhibitors.

### <Experimental Method>

### 1. Cell Culture

The human chondrosarcoma cell line SW-1353 (HTB-94, ATCC, Mansassas, VA, USA) was cultured in DMEM (Dulbecco Modified Eagle's medium) supplemented with 10% FBS (fetal bovine serum) and 1% antibiotic-antifungal agent. An incubator for cell culture was kept at 37°C under 5% CO₂. For cell culture, cells were seeded into a culture flask at a density of 1.2 x 10⁴ cells/cm². When a confluence of 80-90% was reached, the cells were trypsinized. The cells were subcultured 9 times before use in the experiment.

### 2. 3D Culture

SW-1353 cells were grown on a 3D collagen hydrogen with or without a DNA fragment mixture (polydeoxyribonucleotides (PDRN), 450 kDa, sterilized, BR Pharm Co, Wonju, South Korea). SW-1353 cells were harvested from the 2D culture, and the cells in the medium were counted. Next, the cells were spun down and cooled. For use of the DNA fragment mixture as an extracellular matrix (ECM) additive to 3D chondrocytes, 10 mg/ml of the DNA fragment mixture was dissolved in 10X PBS and diluted with 10X PBS at concentrations of 0 µg/ml, 10 µg/ml, 100 µg/ml and 1000 µg/ml. 10X PBS, containing different concentrations of the DNA fragment mixture, and rat tail collagen I solution diluted with DMEM at a concentration of 4 mg/ml, were mixed at a ratio of 1:10, and the mixture was adjusted to a pH of 7.0-7.4 with NaOH. Thereafter, the cells were carefully shaken to avoid air bubbles from entering the collagen solution. 2 µl of the solution was added to each well to construct a 3D gel-cell. The gel was kept in an incubator (37°C and 5% CO₂) for 45 minutes, and the 3D gel-cell matrix was exposed to serum-free medium for 48 hours. For drug screening from the DNA fragment mixture, the DNA fragment mixture was treated with serum-free medium after the SW-1353 cells were sufficiently grown.

### 3. DNA Fragment Mixture Monitoring with Known Criteria Control

SW-1353 cells were treated with 10-1000 µgml of the DNA fragment mixture and 0.1-10 mM of each of glucosamine (GLN), N-acetyl-D-glucosamine (NAG), alliin, sodium butyrate (SB) and chondroitin sulfate (CS) for 24 hours, and stimulated with 10 ng/ml of IL-β for analysis of MMP expression therein.

### 4. Analysis of MMP-3 and MMP-13

To evaluate MMP expression, IL-β was dissolved in serum-free DMEM medium and used at a final concentration of 10 ng/ml. The cells were cultured at 37°C under 5% CO₂. The cell culture supernatant was collected, centrifuged, and then stored at -80°C until use in the experiment. The amounts of MMP-3 and MMP-13 in the culture supernatant were measured using commercially available ELISA (enzyme-linked immunosorbent assays) according to the manufacturer's protocol.

### 5. Bio-staining of Cells in 3D Structure

3D cultured SW-1353 cells were stained with calcein AM, ethidium homodimer 1 (EthD-1) and Hoechst at room temperature for 30 minutes. Thereafter, the cells were washed with PBS, and the cells in PBS were measured using a confocal laser scanning microscope (ZEISS, LSM 710). Images were obtained for each color using ZEN software.

### 6. Measurement of Cell Viability

SW-1353 cells (5 x 10³ cells/well) were cultured in a 96-well plate for 24 hours. The medium was replaced with serum-free media containing various concentrations of the DNA fragment mixture. After 24 hours, 10 µl of CCK-8 was added to each well. After 2 hours of incubation, cell viability was determined by measuring the absorbance at 450 nm by a microplate reader (CLARIOstar, BMG, USA).

### 7. Analysis of Cell Layer Migration

*In vitro* migration analysis was performed using an ibidi Culture-insert. SW-1353 cells were cultured in a Culture-insert dish for 24 hours, and when the cells formed a monolayer, the Culture-insert was removed. To remove the remaining cell debris, the cells were washed twice with medium Thereafter, the monolayered cells were cultured in serum-free medium or serum-free media containing various concentrations of the DNA fragment mixture. Cells in a control group were merely cultured in medium Migration of the cells was measured by imaging with a microscope (EVOS fl, AMG).

### 8. Statistical Analysis

Each value was obtained from an individual sample, and the value is the mean of at least three experiments and expressed as mean ± standard error.

### <Experimental Results>

### 1. 2D Culture Model

### 1.1 Effect of DNA Fragment Mixture on Expression of MMP

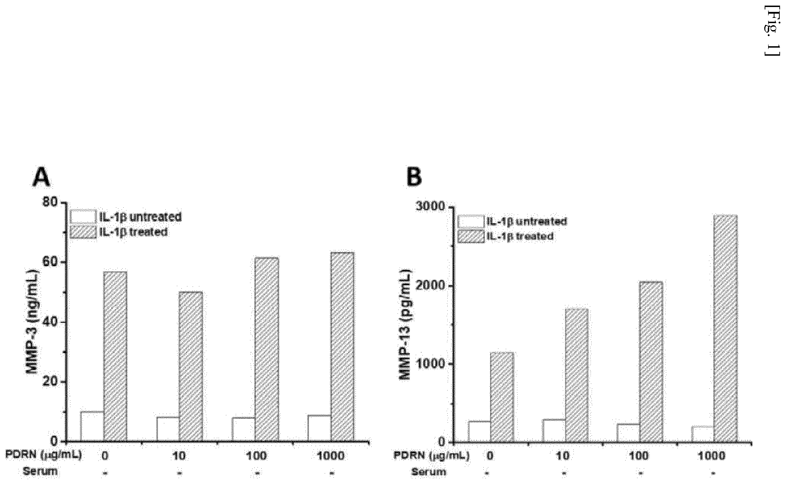

As shown in FIG. 1, SW-1353 cells of a control group were cultured in serum-free media containing the DNA fragment mixture at concentrations of 0, 10, 100 and 1000 µg/ml. Cells of test groups were cultured with 10 ng/ml of IL-β under the same conditions as those of the control group.

As a result, as shown in FIG. 1A, the expression level of MMP-3 increased in the test group treated with IL-β (10 ng/ml) regardless of the concentration of the DNA fragment mixture. However, as shown in FIG. 1B, the expression level of MMP-13 increased as the concentration of the DNA fragment mixture increased.

### 1.2 Change in Migration of SW-1353 Cells by Treatment with DNA Fragment Mixture

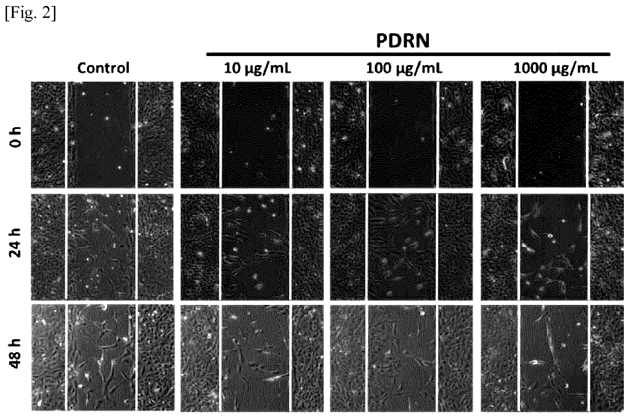

As shown in FIG. 2, in order to examine the role of the DNA fragment mixture in migration of SW-1353 cells, the effect of the DNA fragment mixture on migration of the cells was examined by a 2D wound healing assay. At 0 hr, 24 hr and 48 hr after the SW-1353 cells were treated with 0, 10, 100 and 1000 µg/ml of the DNA fragment mixture, migration of the cells was observed with a microscope.

As a result, migration of the chondrosarcoma cells decreased visibly in the group treated with 1000 µg/ml of the DNA fragment mixture, compared to the control group not containing the DNA fragment mixture.

### 1.3 Effect of DNA Fragment Mixture on Viability of SW-1353 Cells

The most prominent change in arthritis is cartilage deterioration. To evaluate the cartilage protective effect of the DNA fragment mixture, SW-1353 cells were treated with 0, 10, 100 and 1000 µg/ml of the DNA fragment mixture for 2 days, and cell proliferation was evaluated by a CCK-8 assay. It can also evaluate the cytotoxic potential of drugs and toxic materials.

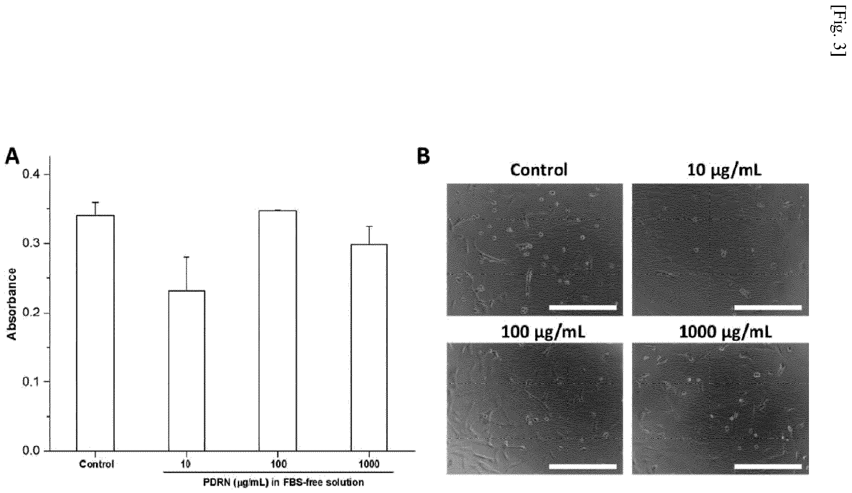

As a result, as shown in FIG. 3, the growth of the SW-1353 cells treated with 10 µgml of the DNA fragment mixture was inhibited compared to that of the control group, and distinct growth of the cells did not appear even at 100 and 1000 µl/ml of the DNA fragment mixture. In addition, living cells were observed by microscopic images, and as a result, it was shown that the DNA fragment mixture was not cytotoxic.

Thus, the DNA fragment mixture exhibited strong anti-proliferative activity depending on the concentration thereof, compared to the control group.

### 1.4 Effect of Glucosamine (GLN) on Expression of MMP

In Example 1.1 above, it was found that the DNA fragment mixture induced the production of MMP-3 and MMP-13. For this reason, glucosamine (GLN) that inhibits MMP production was added together with the DNA fragment mixture, and whether MMP-3 and MMP-13 would be produced was tested.

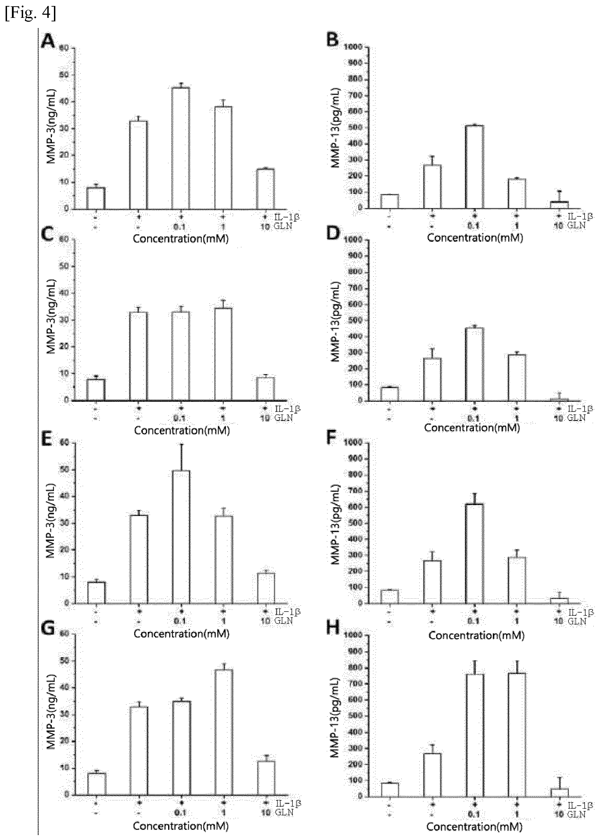

As shown in FIG. 4, SW-1353 cells were treated with 0, 10, 100 and 1000 µl/ml of the DNA fragment mixture and 0, 0.1, 1 and 10 mM of glucosamine (GLN), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of glucosamine (GLN), the expression levels of MMP-3 and MMP-13 proteins decreased regardless of the concentration of the DNA fragment mixture.

### 1.5 Effect of N-Acetyl-D-Glucosamine (NAG) on Expression of MMP

In Example 1.1 above, it was found that the DNA fragment mixture induced the production of MMP-3 and MMP-13. For this reason, N-acetyl-D-glucosamine (NAG) that inhibits MMP production was added together with the DNA fragment mixture, and whether MMP-3 and MMP-13 would be produced was tested.

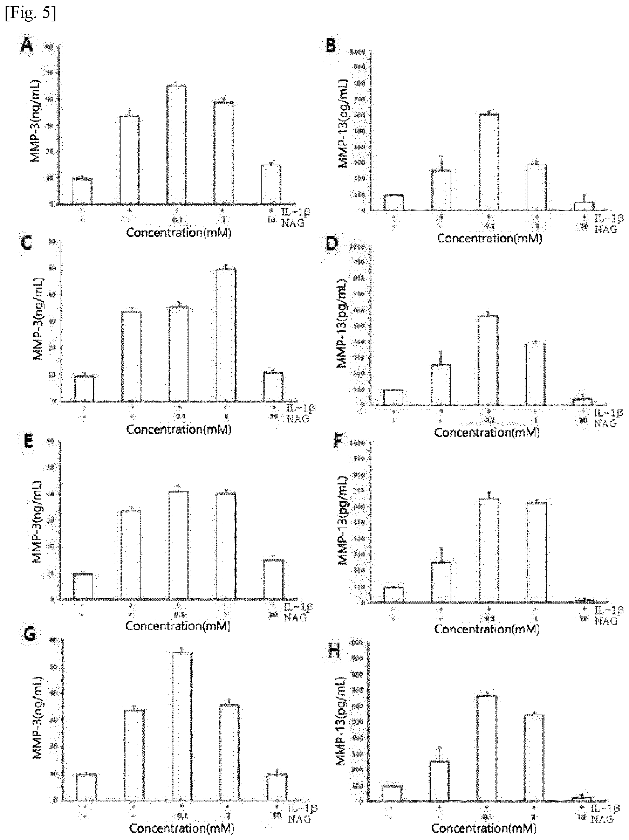

As shown in FIG. 5, SW-1353 cells were treated with 0, 10, 100 and 1000 µl/ml of the DNA fragment mixture and 0, 0.1, 1 and 10 mM of N-acetyl-D-glucosamine (NAG), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of N-acetyl-D-glucosamine (NAG), the expression levels of MMP-3 and MMP-13 proteins decreased regardless of the concentration of the DNA fragment mixture.

### 1.6 Effect of Alliin on Expression of MMP

In Example 1.1 above, it was found that the DNA fragment mixture induced the production of MMP-3 and MMP-13. For this reason, alliin that inhibits MMP production was added together with the DNA fragment mixture, and whether MMP-3 and MMP-13 would be produced was tested.

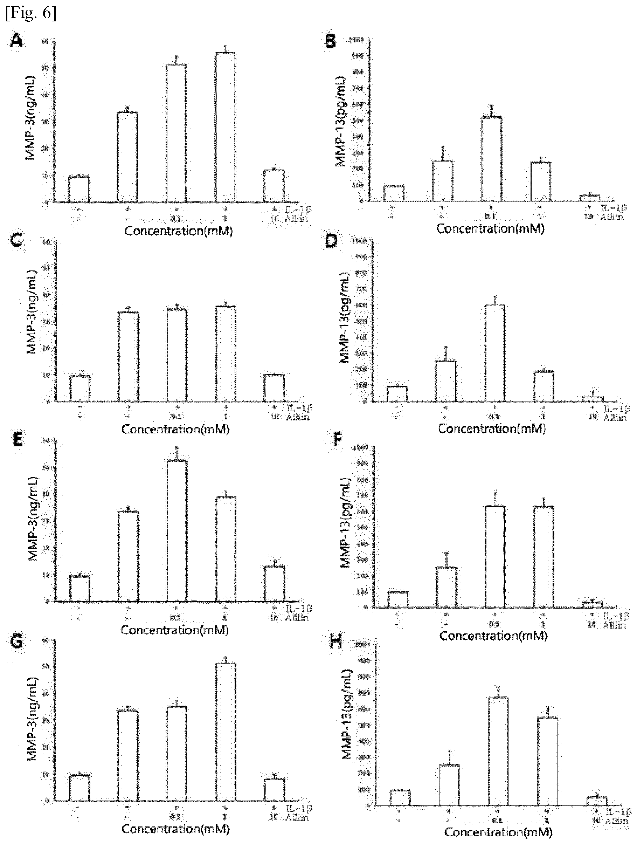

As shown in FIG. 6, SW-1353 cells were treated with 0, 10, 100 and 1000 µl/ml of the DNA fragment mixture and 0, 0.1, 1 and 10 mM of alliin, and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of alliin, the expression levels of MMP-3 and MMP-13 proteins decreased regardless of the concentration of the DNA fragment mixture.

### 1.7 Effect of Sodium Butyrate (SB) on Expression of MMP

In Example 1.1 above, it was found that the DNA fragment mixture induced the production of MMP-3 and MMP-13. For this reason, sodium butyrate (SB) that inhibits MMP production was added together with the DNA fragment mixture, and whether MMP-3 and MMP-13 would be produced was tested.

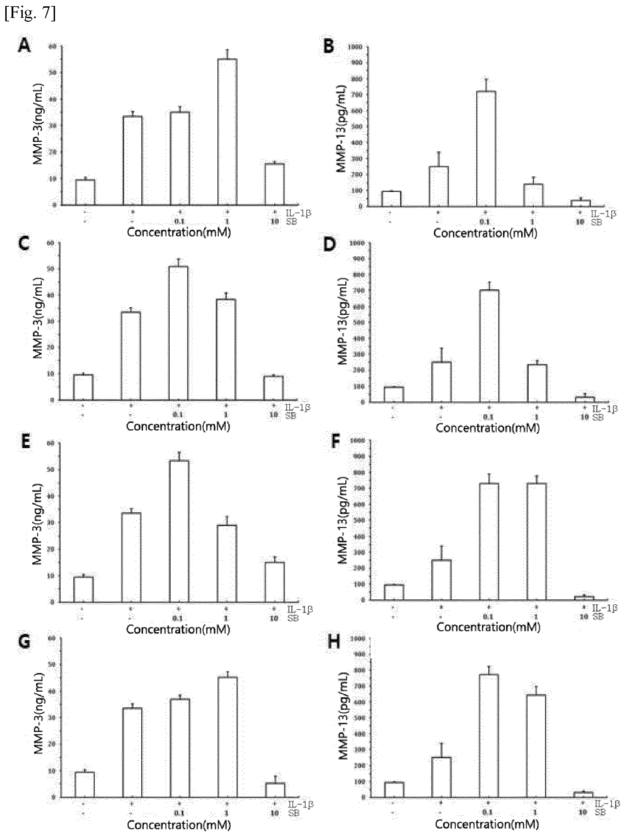

As shown in FIG. 7, SW-1353 cells were treated with 0, 10, 100 and 1000 µl/ml of the DNA fragment mixture and 0, 0.1, 1 and 10 mM of sodium butyrate (SB), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of sodium butyrate (SB), the expression levels of MMP-3 and MMP-13 proteins decreased regardless of the concentration of the DNA fragment mixture.

### 1.8 Effect of Chondroitin Sulfate (CS) on Expression of MMP

In Example 1.1 above, it was found that the DNA fragment mixture induced the production of MMP-3 and MMP-13. For this reason, chondroitin sulfate (CS) that inhibits MMP production was added together with the DNA fragment mixture, and whether MMP-3 and MMP-13 would be produced was tested.

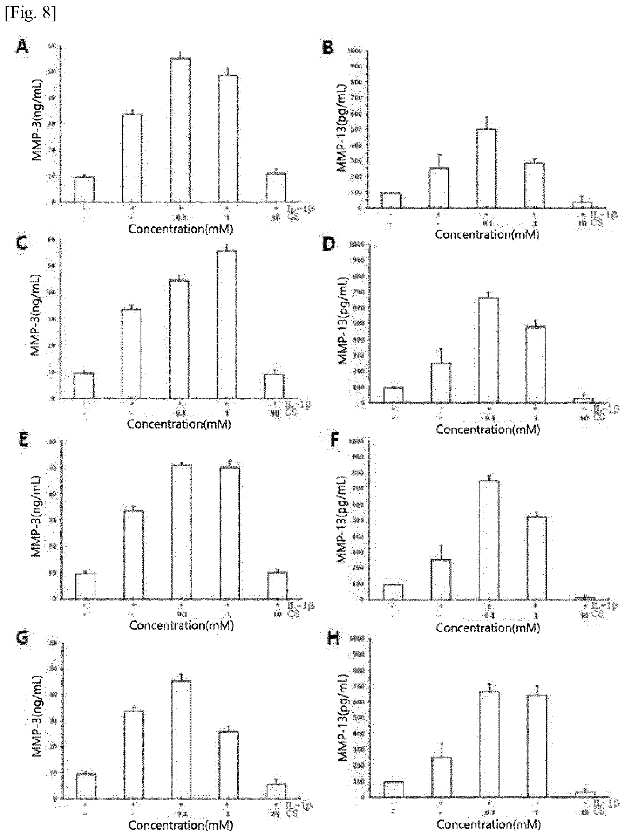

As shown in FIG. 8, SW-1353 cells were treated with 0, 10, 100 and 1000 µl/ml of the DNA fragment mixture and 0,0.1, 1 and 10 mM of chondroitin sulfate (CS), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of chondroitin sulfate (CS), the expression levels of MMP-3 and MMP-13 proteins decreased regardless of the concentration of the DNA fragment mixture.

### 2.3D Culture Model

### 2.13D Cartilage Construction by SW-1353 Cell Network

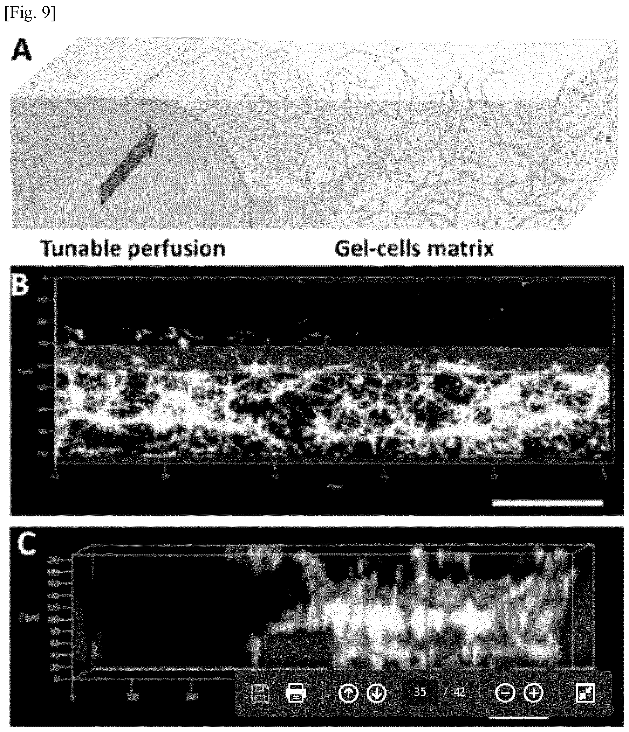

To construct a 3D cartilage chip including a collagen-based extracellular matrix (ECM), a 3D cartilage *in vitro* model with a microfluidic platform was constructed in which a gel-cell matrix and a tunable-dynamic flow tube were spaced apart from each other (FIG. 9A). It was configured such that a medium for cell culture would flow in the tunable tube and the medium would be perfused toward the opposite side of the gel-cell matrix at an average flow rate of the gel-cell matrix. In addition, SW-1353 cells were cultured in the collagen matrix in a 3D dynamic fashion. During several days when the SW-1353 cells were exposed to the medium, it was observed that the SW-1353 cells had a morphology like that of fibroblasts as shown in FIG. 9B. In addition, as shown in FIG. 9C, it was confirmed that the 3D cartilage chip of SW-1353 chondrosarcoma was well constructed.

### 2.2 Morphology of SW-1353 Cells in 3D Dynamic Culture Model

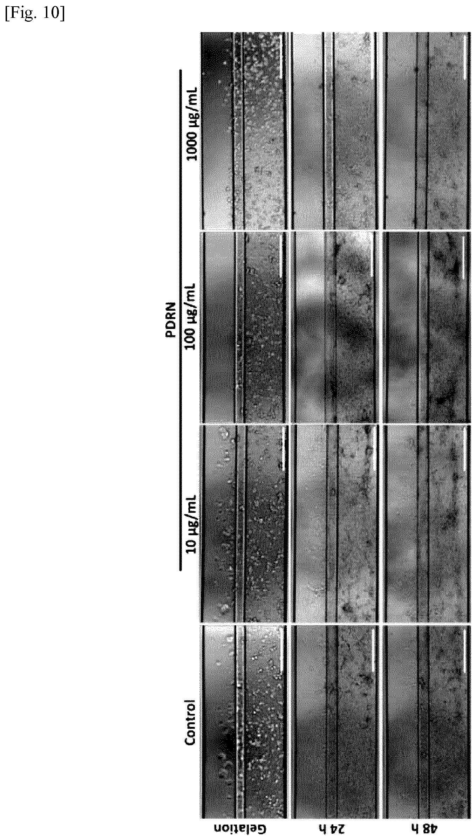

FIG. 10 shows images of cells cultured with various concentrations of the DNA fragment mixture during 48 hours of 3D dynamic culture. Each gel-cell matrix included different concentrations (0 (control), 10, 100 and 1000 µg/ml) on 4 mg/ml of a collagen matrix. After 45 minutes of gelation, images after 24 hours and 48 hours were measured, and as a result, it was shown that SW-1353 was well seeded onto the gel.

100 µl of serum-free medium was added to each tube, and SW-1353 cells were cultured on ECM gel matrices including different concentrations of the DNA fragment mixture in a 3D dynamic fashion. After exposure to the medium, aggregation of the cells was not observed for 48 hours, and a morphology like that of fibroblasts was formed on the gel surface. Thus, it was confirmed that the DNA fragment had no effect on the arrangement of the cells on the gel.

### 2.3 Effect of DNA Fragment Mixture on Viability of SW-1353 Cells in 3D Dynamic Culture Model

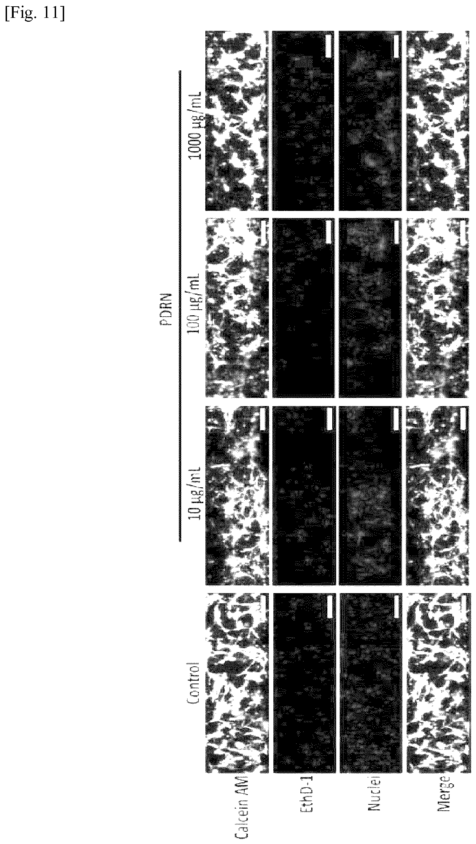

FIG. 11 shows cell viability in the 3D dynamic culture environment shown in FIG. 10. To reveal the effect of the DNA fragment mixture on the viability of SW-1353 cells, the measurement of cell viability in 3D culture was performed at 24 hours after the cells were treated with 0 (control), 10, 100 and 1000 µg/ml of the DNA fragment mixture ECM in the presence or absence of IL-β. In comparison with an untreated control group, 10-1000 µg/ml of the DNA fragment mixture ECM alone showed no particular cytotoxic effect against the SW-1353 cells. In the presence of IL-β, different concentrations of DNA fragment mixture ECM also showed no cytotoxic effect (FIG. 11).

### 2.4 Effect of DNA Fragment Mixture on Expression of MMP in 3D Dynamic Culture Model

During 3D cell culture, chondrocytes were cultured in 10% serum-containing medium or serum-free medium, and the cultured chondrocytes were stimulated with IL-β (10 ng/ml).

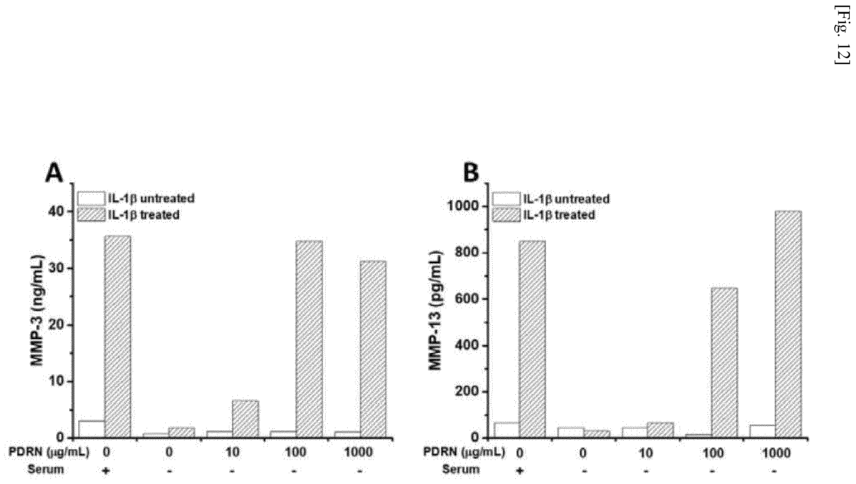

As a result, as shown in FIG. 12, the expression of MMP-1 and MMP-13 in 10% serum-containing medium was similar to the expression of MMP in the presence of 100 and 1000 µg/ml of the DNA fragment mixture. Thus, it was confirmed that a high concentration of the DNA fragment mixture could worsen arthritis by activating MMP.

In addition, after 24 hours of treatment with the DNA fragment mixture and IL-β in serum-free medium, two markers (MMP-3 and MMP-13) which are frequently involved in degenerative arthritis disease were analyzed.

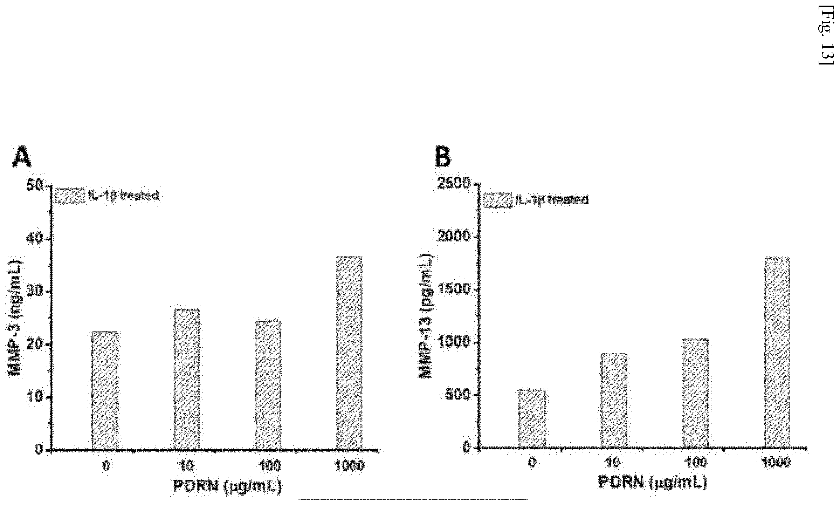

As a result, as shown in FIG. 13, the expression level of MMP-13 in the presence of the DNA fragment mixture increased depending on the concentration of the DNA fragment mixture. Thus, it was confirmed that the DNA fragment mixture could worsen arthritis by inducing the production of MMP.

### 2.5 Effect of Glucosamine (GLN) on Expression of MMP

In Example 2.4 above, it was confirmed that the DNA fragment mixture induced the production of MMP-3 and MMP-13. For this reason, glucosamine (GLN) that inhibits MMP production was added together with the DNA fragment mixture, and whether MMP-3 and MMP-13 would be produced was tested.

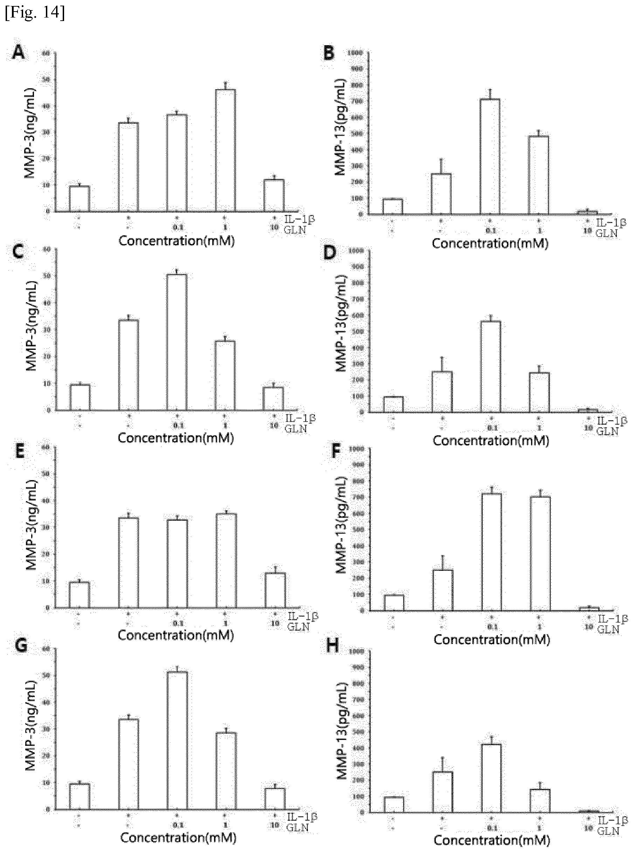

As shown in FIG. 14, SW-1353 cells were treated with 0, 10, 100 and 1000 µl/ml of the DNA fragment mixture and 0, 0.1, 1 and 10 mM of glucosamine (GLN), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of glucosamine (GLN), the expression levels of MMP-3 and MMP-13 proteins decreased regardless of the concentration of the DNA fragment mixture.

### 2.6 Effect of N-Acetyl-D-Glucosamine (NAG) on Expression of MMP

In Example 2.4 above, it was confirmed that the DNA fragment mixture induced the production of MMP-3 and MMP-13. For this reason, N-acetyl-D-glucosamine (NAG) that inhibits MMP production was added together with the DNA fragment mixture, and whether MMP-3 and MMP-13 would be produced was tested.

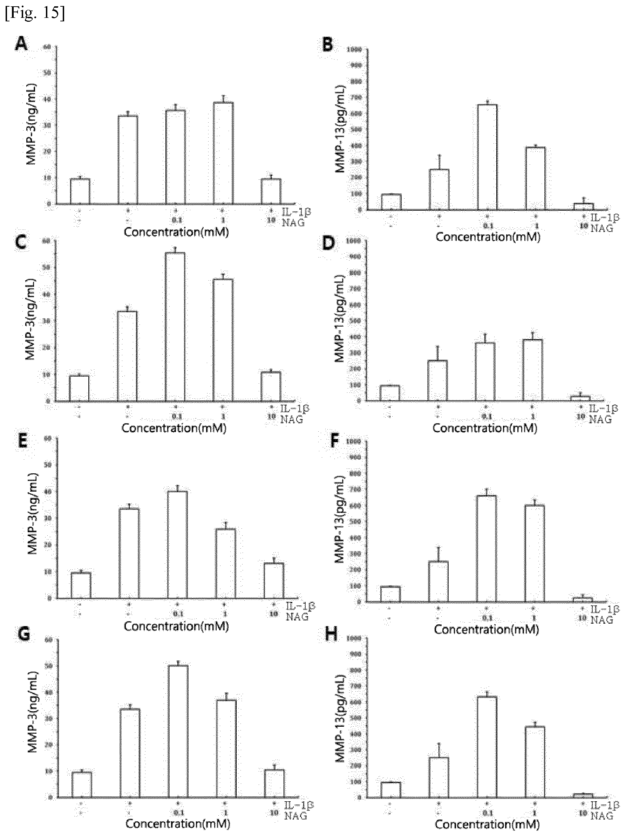

As shown in FIG. 15, SW-1353 cells were treated with 0, 10, 100 and 1000 µl/ml of the DNA fragment mixture and 0, 0.1, 1 and 10 mM of N-acetyl-D-glucosamine (NAG), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of N-acetyl-D-glucosamine (NAG), the expression levels of MMP-3 and MMP-13 proteins decreased regardless of the concentration of the DNA fragment mixture.

### 2.7 Effect of Alliin on Expression of MMP

In Example 2.4 above, it was confirmed that the DNA fragment mixture induced the production of MMP-3 and MMP-13. For this reason, alliin that inhibits MMP production was added together with the DNA fragment mixture, and whether MMP-3 and MMP-13 would be produced was tested.

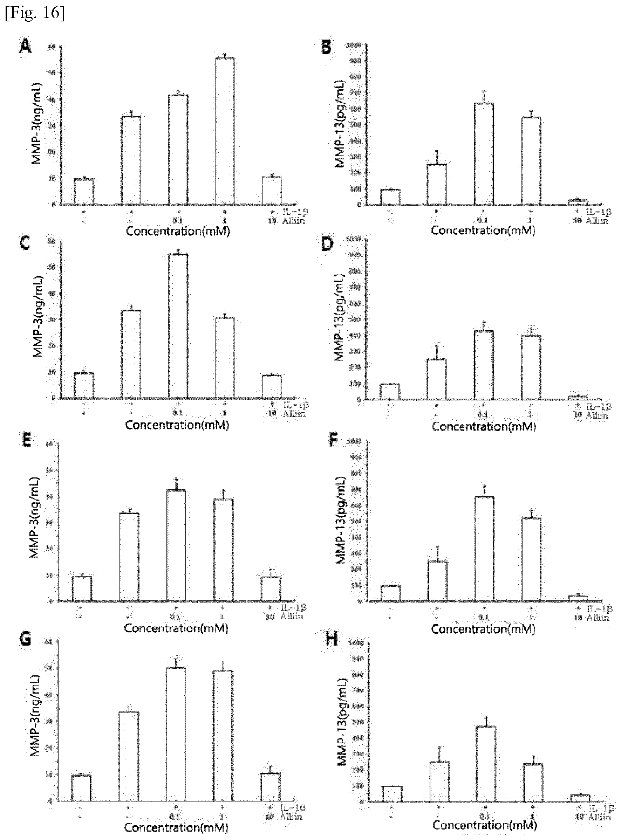

As shown in FIG. 16, SW-1353 cells were treated with 0, 10, 100 and 1000 µl/ml of the DNA fragment mixture and 0, 0.1, 1 and 10 mM of alliin, and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of alliin, the expression levels of MMP-3 and MMP-13 proteins decreased regardless of the concentration of the DNA fragment mixture.

### 2.8 Effect of Sodium Butyrate (SB) on Expression of MMP

In Example 2.4 above, it was confirmed that the DNA fragment mixture induced the production of MMP-3 and MMP-13. For this reason, sodium butyrate (SB) that inhibits MMP production was added together with the DNA fragment mixture, and whether MMP-3 and MMP-13 would be produced was tested.

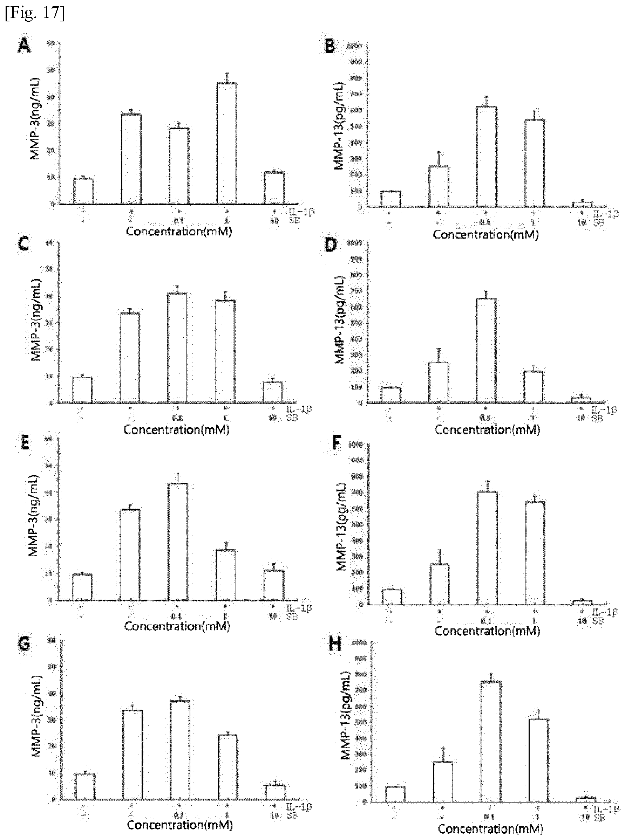

As shown in FIG. 17, SW-1353 cells were treated with 0, 10, 100 and 1000 µl/ml of the DNA fragment mixture and 0, 0.1, 1 and 10 mM of sodium butyrate (SB), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of sodium butyrate (SB), the expression levels of MMP-3 and MMP-13 proteins decreased regardless of the concentration of the DNA fragment mixture.

### 2.9 Effect of Chondroitin Sulfate (CS) on Expression of MMP

In Example 2.4 above, it was confirmed that the DNA fragment mixture induced the production of MMP-3 and MMP-13. For this reason, chondroitin sulfate (CS) that inhibits MMP production was added together with the DNA fragment mixture, and whether MMP-3 and MMP-13 would be produced was tested.

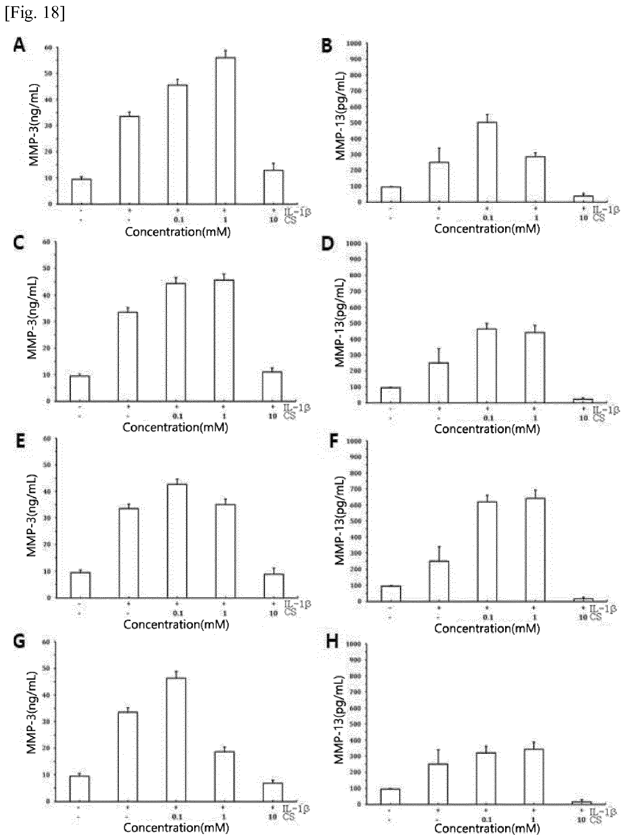

As shown in FIG. 18, SW-1353 cells were treated with 0, 10, 100 and 1000 µl/ml of the DNA fragment mixture and 0,0.1,1 and 10 mM of chondroitin sulfate (CS), and 10 ng/ml of IL-β was added thereto. Next, the expression levels of MMP-3 and MMP-13 in the treated groups were analyzed in comparison with those in an untreated control group.

As a result, it was shown that when the cells were treated with 10 mM of chondroitin sulfate (CS), the expression levels of MMP-3 and MMP-13 proteins decreased regardless of the concentration of the DNA fragment mixture.

### <Conclusion>

For drug screening research related to degenerative arthritis or rheumatoid arthritis, a 3D in vitro cartilage model was constructed. It was found that the DNA fragment mixture (PDRN) as a 3D matrix would play an important role in osteoarthritis treatment.

Specifically, cell migration was significantly inhibited by PDRN. This demonstrates that the DNA fragment mixture (PDRN) exhibits an effect on the treatment of arthritis by significantly inhibiting cell invasion and metastasis.

However, it was also found that the DNA fragment mixture (PDRN) has the problem of inducing the production of MMP, which can adversely affect the treatment of arthritis. It was also demonstrated that this problem could be overcome by using the DNA fragment together with any one selected from the group consisting of glucosamine, N-acetyl-D-glucosamine (NAG), alliin, sodium butyrate (SB) and chondroitin sulfate (CS), which are MMP production inhibitors.

Therefore, the present invention may provide a composition which is capable of exhibiting the effect of preventing and treating arthritis while inhibiting MMP production induced by the DNA fragment mixture (PDRN), by including, as active ingredients, the DNA fragment mixture (PDRN) together with an MMP production inhibitor, for example, any one selected from the group consisting of glucosamine or a pharmaceutically acceptable salt thereof, N-acetyl-D-glucosamine (NAG), alliin, sodium butyrate (SB) and chondroitin sulfate (CS).

As described above, the present invention provides a composition for preventing and treating arthritis, which can inhibit matrix metalloproteinase production induced by the DNA fragment mixture, and, thus, can be effectively used without adverse effects for induction of chondrocyte proliferation, repair of damaged chondrocytes, regeneration of cartilage, and relief of arthritis.

Although the specific embodiments of the present invention have been disclosed for illustrative purposes, those skilled in the art will appreciate that various modifications, additions and substitutions are possible without departing from the scope and spirit of the invention as disclosed in the accompanying claims.

## Claims

1. A composition for preventing and treating arthritis, comprising a DNA fragment mixture and a matrix metalloproteinase production (MMP) inhibitor.

2. The composition of claim 1, wherein the MMP production inhibitor is glucosamine or a pharmaceutically acceptable salt thereof

3. The composition of claim 1, wherein the MMP production inhibitor is N-acetyl-D-glucosamine (NAG) or a pharmaceutically acceptable salt thereof

4. The composition of claim 1, wherein the MMP production inhibitor is alliin or a pharmaceutically acceptable salt thereof.

5. The composition of claim 1, wherein the MMP production inhibitor is sodium butyrate (SB).

6. The composition of claim 1, wherein the MMP production inhibitor is chondroitin sulfate (CS).
